# EUROPÄISCHE PATENTSCHRIFT

(11) **EP 0 985 422 B1**
(45) Veröffentlichungstag und Bekanntmachung des Hinweises auf die Patenterteilung: **01.08.2007**
(21) Anmeldenummer: 99116227.2
(22) Anmeldetag: 17.08.1999
(51) Int. Cl.: A61M 16/08

(54) **Feuchtigkeits- und Bakterienbarriere für medizinische Komponenten**
Humidity and bacterial protection for medical components
Protection contre l'humidité et les bactéries pour des composants médicaux

(30) Priorität: 09.09.1998 SE 9803047
(43) Veröffentlichungstag der Anmeldung: 15.03.2000
(73) Patentinhaber: Maquet Critical Care AB, 171 95 Solna (SE)
(72) Erfinder: Skog, Göran, 168 56 Bromma (SE); Wallén, Lars, 163 47 Spanga (SE); Arvidsson, Carl-Erik, 171 39 Solna (SE)
(74) Vertreter: Stein, Jan Anders Lennart

(56) Entgegenhaltungen:
- EP-A- 0 510 377
- WO-A-86/02820
- US-A- 4 558 708
- US-A- 5 789 660

## Beschreibung

Die Erfindung bezieht sich auf eine Feuchtigkeits- und Bakterienbarriere für medizinische Komponenten, die im Anschluß an ein Meßrohr, das in den Flußwegen eines Beatmungsgerätes angeordnet ist, angebracht ist.

Damit ein Beatmungsgerät überwacht und gesteuert werden kann, werden mit Hilfe von medizinischen Komponenten eine Anzahl Parameter, vorzugsweise an der Exspirationsseite, gemessen. Die medizinischen Komponenten sind vorzugsweise in einer Einheit, die an dem genannten Meßrohr angeschlossen wird, fest angeordnet. Um die medizinischen Komponenten von "Verunreinigungen" in der Atmungsluft wie Arztneimitteln und Körperflüssigkeiten zu schützen, ist es bekannt, im Anschluß am Eingang des Meßrohres eine Feuchtigkeits- und Bakterienbarriere in Form eines Filters, das in einem Mundstück aus Kunststoff angeordnet ist, anzubringen. Auf diese Weise kann ein Autoklavieren der meistens empfindlichen medizinischen Komponenten vermieden werden. Das Mundstück aus Kunststoff und das Filter sind meistens aus einem Einwegmaterial, das nach jeder Verwendung verworfen wird und mit einem neuen Mundstück mit Filter ersetzt wird, wenn ein neuer Patient an dem Beatmungsgerät angeschlossen wird. Dieses Einwegprodukt bringt für das Krankenhaus relativ große Kosten mitsich und ist außerdem eine Belastung für die Umwelt. Außerdem kann das Filter einen gewissen Flußwiderstand in der Exspirationsleitung mitsichbringen. Wenn das Filter eine längere Zeit benutzt wird, kann es verstopft werden, was einen zu hohen Flußwiderstand mitsichbringt. Dies kann wiederum zu einem erhöhten Druck in der Lunge führen, was ein erhöhtes Risiko für Schäden mitsichbringt.

In der EP 0 841 083 ist versucht worden, dieses Problem zu lösen, indem hier ein Verfahren und eine Anordnung zur Funktionskontrolle von Filtern geschaffen worden ist, um somit die Benutzungszeit zu optimieren, ohne zu riskieren, daß der Patient Probleme bekommt.

Der Erfindung liegt die Aufgabe zugrunde, eine verhältnismäßig einfache und billige austauschbare Feuchtigkeits- und Bakterienbarriere für medizinische Komponenten zu erhalten, die keinen Flußwiderstand in den Flußwegen des Beatmungsgerätes verursacht.

Diese Aufgabe ist erfindungsgemäß durch einen Träger nach Anspruch 1 gelöst. Hierdurch wird am Eingang des Meßrohres ein Filter vermieden mit den Nachteilen, die diese Applizierung mitsichbringt. Durch die Erfindung kann jede im Anschluß an das Meßrohr applizierte medizinische Komponente eine individuell angepaßte Barriere erhalten. Die Barrieren können bei Bedarf dicht an der Komponente oder mit Abstand zur Komponente appliziert werden. Dadurch, daß die Barrieren an einem gemeinsamen Träger appliziert sind, können sie bei Bedarf rasch und einfach ausgewechselt werden. Da ein Träger dieser Art im Aufbau verhältnismäßig einfach ist, können die Herstellungskosten niedrig gehalten werden.

In der US-PS 5 419 326 ist ein Ultraschallflußmesser beschrieben, in dem der Sender und der Empfänger mit Abstand voneinander entlang einer Meßstrecke, die schräg im Vergleich zur Achse einer rohrförmigen Meßkammer verläuft, angeordnet sind. Um dabei die Hygiene sicherzustellen, wird in Verbindung mit jedem neuen Patienten ein steriles Einsatzrohr in die Meßkammer eingeführt. Das sterile Rohr ist mit Meßfenstern versehen, die so angepaßt sind, daß sie über den Öffnungen in der Meßkammer liegen. In den Meßfenstern sind Membranen angeordnet, die für Ultraschallsignale durchlässig, aber für Feuchtigkeit und Bakterien undurchlässig sind, so daß die Ultraschallsignale entlang der Meßstrecke durch das sterile Einsatzrohr passieren können. Hierdurch wird vermieden, daß das Krankenhauspersonal nach jeder Verwendung den Flußmesser autoklavieren muß, da insbesondere die Ultraschallsende- und Empfangseinheit empfindliche Teile in dem Flußmesser sind. Die Membranen sind mit Abstand zu den Ultraschallsende- und Empfangsköpfen angeordnet. Damit die Ultraschallsignale die Sende- bzw. Empfangseinheit erreichen können, müssen die Signale zunächst die Membran und danach einen verhältnismäßig großen Luftspalt passieren. Dieser Übergang von einer Membran zu einem verhältnismäßig großen Luftspalt kann zu einer hohen akustischen Impedanz, d.h. einer hohen Schallwellenreflexion führen. Dies kann zu verhältnismäßig großen akustischen Verlusten führen, so daß ein nicht annehmbar niedriges Schallsignal die Sende- bzw. Empfangsköpfe erreicht. Die Lösung dieses Problems ist es, u.a. die Membran dicht an den Ultraschallsende- und Empfangsköpfen anzubringen. Bei einer solchen Lösung, die in der noch nicht veröffentlichten schwedischen Patentanmeldung 9801007 beschrieben worden ist, sind die Membranen nicht mehr an einem sterilen Einsatzrohr angebracht, sondern werden direkt an den Sende- und Empfangsköpfen befestigt.

US 5,789,660 beschreibt einen Aternwegsadapter mit Feuchtigkeits- und Bakterienbarrieren zur Aufzeichnung der CO₂-Konzentration in Aternluft. Der Adapter beinhaltet eine verbesserte Sensorkonfiguration, weiche Toträume in dem Adapter minimiert. Besagter Adapter ist jedoch nur für eine einzige medizinische Komponente ausgelegt, sodass, wenn mehrere medizinische Komponenten vorhanden sind, der Austausch der Feuchtigkeits- und Bakterienbarrieren aufwendig ist.

Es gibt andere medizinische Komponenten mit den Forderungen, daß die Membran oder das Filter dicht an der jeweiligen Komponente angebracht werden soll, damit das bestmögliche Meßergebnis erhalten wird. Da der Träger für die Barriere beim Gegenstand der US-Schrift in die Meßkammer einschiebbar und placierbar ist, kann es aufgrund der Wandstärke der Meßkammer schwierig sein, die Barriere dicht an einer medizinischen Komponente anzubringen. In der US-Schrift sind zwar zwei Barrieren am sterilen Rohr angebracht, aber diese zwei Barrieren sind lediglich für eine einzige medizinische Komponente vorgesehen.

Bei einem Beatmungsgerät mit einem Meßrohr und einer Einheit der eingangs genannten Art, in der fest montierte Komponenten angeordnet sind, ist der Träger nach der Erfindung zwischen dem Meßrohr und der Einheit anbringbar. Durch die Placierung des Trägers können bei Bearf die genannten Barrieren dicht an einer Komponente angebracht werden.

Da die Komponenten in der Einheit in der Regel in einer Reihe nacheinander angeordnet sind, weist der Träger vorzugsweise eine langgestreckte Form auf. Das Querschnittprofil des Trägers ist vorzugsweise an das Außenprofil des Meßrohres angepaßt. Der Träger ist vorzugsweise aus einem Kunststoffmaterial hergestellt.

In einer vorteilhaften Weiterentwicklung der Erfindung wird vorgeschlagen, daß die Peripherieoberfläche des Trägers mit einer Dichtung versehen ist, die bei einem applizierten Träger gegen das Meßrohr anliegbar ist. Hierdurch wird sichergestellt, daß kein Gas aus dem Meßrohr heraussickert.

Die Barrieren nach der Erfindung Filter und/oder Membranen.

Wie bereits erwähnt, ist der Träger nach der Erfindung vorzugsweise austauschbar. Hierdurch kann vor jedem neuen Patienten ein neuer Träger angebracht werden.

Die Erfindung ist nachfolgend anhand eines in den Zeichnungen dargestellten Ausführungsbeispiels näher erläutert. Es zeigen:
- FIG. 1: ein schematsich gezeichnetes Beatmungsgerät mit einem Meßrohr und einer Einheit für medizinische Komponenten, die im Anschluß an der Exspirationsleitung angeordnet sind,
- FIG. 2: ein Meßrohr in einem Längsschnitt, eine Einheit mit schematisch gezeichneten medizinischen Komponenten und dazwischen einen Träger für Feuchtigkeits- und Bakterienbarriere nach der Erfindung und
- FIG. 3: einen Träger nach FIG. 2 in einer Draufsicht.

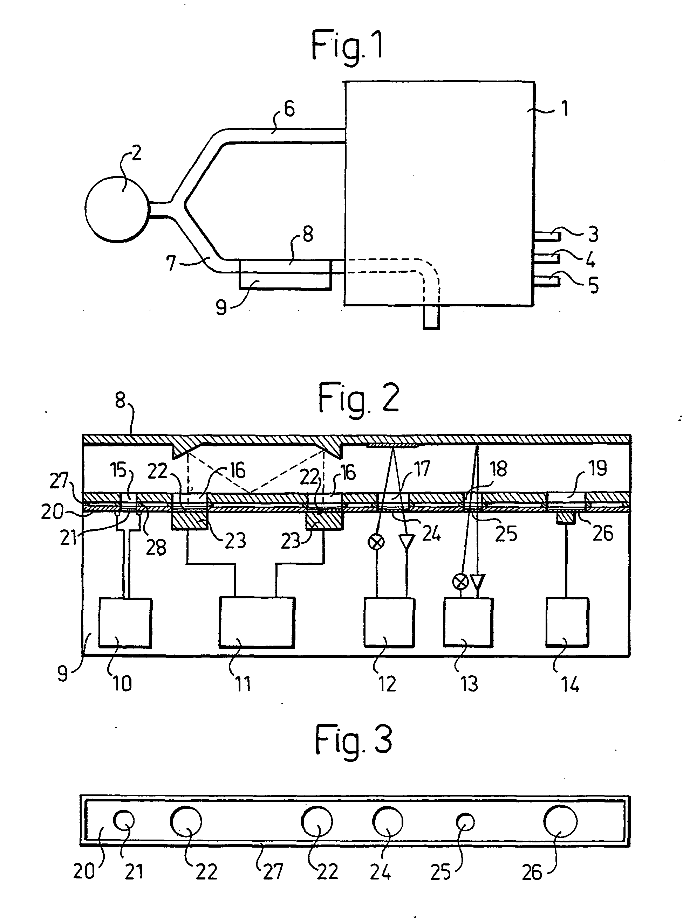

In der FIG. 1 ist ein Beatmungsgerät 1 gezeigt, an dem ein Patient 2 angeschlossen ist, um somit Atmungsgas zugeführt zu bekommen. Diejenigen Gase, die zusammen das Atmungsgas bilden, werden dem Beatmungsgerät 1 über Gasanschlüsse 3, 4 und 5 zugeführt. Bei der Einatmung wird das Atmungsgas über eine Inspirationsleitung 6 dem Patienten 2 zugeleitet. Bei der Ausatmung wird das Atmungsgas vom Patienten 2 durch eine Exspirationsleitung 7 und über das Beatmungsgerät 1 in die Umgebung, meistens an einen hier nicht gezeigten Sammelbehälter, geführt. In der Exspirationsleitung 7 ist ein Meßrohr 8 angeordnet, das mit einer Einheit 9, beinhaltend medizinische Komponenten, verbunden ist.

In der FIG. 2 ist das Meßrohr 8 und die Einheit 9 in einem im Vergleich zur FIG. 1 größeren Maßstab gezeigt. Die medizinischen Komponenten sind in der Einheit 9 fest angeordnet. In diesem Ausführungsbeispiel umfaßt die Einheit 9 einen Druckgeber 10, einen Ultraschallflußmesser 11, einen Kohlendioxydmesser 12, einen Sauerstoffsensor 13 und einen Temperaturmesser 14. Die Komponenten sind in einer Linie nacheinander angeordnet. Die Komponenten 10 bis 14 sind mit dem Inneren des Meßrohres 8 über Öffnungen 15 bis 19 in der Wand des Meßrohres 8 verbunden. Um zu vermeiden, daß die Komponenten 10 bis 14 durch die "verunreinigte" Ausatmungsluft kontaminiert werden, sind zwischen dem Meßrohr 8 und den Komponenten 10 bis 14 Feuchtigkeits- und Bakterienbarrieren angeordnet. Die Barrieren sind auf einem gemeinsamen Träger 20, z.B. in Form einer Scheibe aus Kunststoff, derart angebracht, daß jede Barriere genau vor jeweils einer Komponente 10 bis 14 angeordnet ist. So ist die Barriere für den Druckgeber 10 ein Bakterienfilter 21, für den Ultraschallflußmesser 11 Folien 22, die vorzugsweise dicht an den Meßköpfen 23 anliegen, für den Kohlendioxydmesser 12 ein Fenster 24, für den Sauerstoffsensor 13 eine transparente Folie 25 und für den Temperaturmesser 14 eine Metallfolie 26.

Beim Auswechseln des Trägers 20 wird vorzugsweise die Einheit 9 vom Meßrohr 8 entfernt. Danach wird der Träger 20 mit dessen Barrieren 21, 22, 24, 25, 26 vom Meßrohr 8 entfernt und gegen einen neuen für den Zweck passenden Träger 20 ausgewechselt. Danach wird die Einheit 9 mit den medizinischen Komponenten 10 bis 14 wieder an ihrem Platz appliziert. Die Peripherieoberfläche des Trägers 20 ist vorzugsweise mit einer Dichtung 27 versehen, die bei einem applizierten Träger 20 gegen das Meßrohr 8 anliegt. Die Dichtung 27 soll verhindern, daß Gas aus dem Meßrohr heraussickert. In diesem Ausführungsbeispiel sind auch Dichtungsringe um die Öffnungen 15 bis 19 angeordnet. In dieser FIG. hat lediglich ein Dichtungsring ein Bezugszeichen 28, und zwar für die Öffnung 15, erhalten.

FIG. 3, die eine Draufsicht des Trägers 20 ist, zeigt, daß die Barrieren 21, 22, 24, 25, 26 in einer Reihe nacheinander angeordnet sind. In der FIG. ist auch die Dichtung 27 gezeigt, die entlang der Peripherieoberfläche des Trägers 20 angeordnet ist.

Im Rahmen der Erfindung können selbstverständlich mehr oder weniger medizinische Komponenten als die hier erwähnten, in der Einheit appliziert werden. Es ist ferner selbstverständlich nicht notwendig, daß diese in einer Reihe nacheinander angeordnet sein müssen, wie das Beispiel es zeigt. Das Wesentliche ist, einen austauschbaren Träger nach der Erfindung mit Feuchtigkeits- und Bakterienbarrieren zu erhalten, die so angeordnet sind, daß jede, bei einem am Beatmungsgerät applizierten Träger, genau vor jeweils einer Komponente angeordnet ist. Die Barrieren können durch die Applizierung des Trägers zwischen dem Meßrohr und der Einheit bei Bedarf vorzugsweise dicht an der jeweiligen medizinischen Komponente angebracht werden.

Durch die Erfindung wird vermieden, daß ein Flußwiderstand in dem Exspirationsflußweg des Beatmungsgerätes entsteht. Ein weiterer wesentlicher Vorteil der Erfindung ist es, daß durch den für die Barrieren gemeinsamen Träger, die Barriere für sämtliche medizinische Komponenten auf einfache Weise gleichzeitig ausgetauscht werden können.

## Patentansprüche

1. Träger (20) mit Feuchtigkeits- und Bakterienbarrieren (21, 22, 24, 25, 26) für eine Mehrzahl von medizinischen Komponenten (10 bis 14), zum Anschluß an ein Meßrohr (8), das in den Flußwegen eines Beatmungsgerätes (1) angeordnet ist, **dadurch gekennzeichnet, daß** auf dem Träger (20) mindestens zwei Feuchtigkeits- und Bakterienbarrieren (21, 22, 24, 25, 26) derart angebracht sind, daß diese jeweils, bei einem in dem Beatmungsgerät (1) applizierten Träger (20) genau vor jeder Komponente (10 bis 14) angeordnet werden können.

2. Träger (20) für Feuchtigkeits- und Bakterienbarrieren nach Anspruch 1, **dadurch gekennzeichnet, daß** der Träger (20) eine langgestreckte Form aufweist.

3. Träger (20) für Feuchtigkeits- und Bakterienbarrieren nach Anspruch 1 oder 2, **dadurch gekennzeichnet, daß** die Peripherieoberfläche des Trägers (20) mit einer Dichtung (27) versehen ist, die bei einem applizierten Träger (20) gegen das Meßrohr (8) anliegt.

4. Träger (20) für Feuchtigkeits- und Bakterienbarrieren nach einem der Ansprüche 1 bis 3, **dadurch gekennzeichnet, daß** die Barrieren (21, 22, 24, 25, 26) Filter und/oder Membranen sind.

5. Beatmungsgerät (1) umfassend ein Meßrohr (8) und eine Einheit (9), in der fest montierte Komponenten (10 bis 14) angeordnet sind, wobei ein Träger (20) nach einem der Ansprüche 1 bis 4 zwischen dem Meßrohr (8) und der Einheit (9) angebracht ist.

6. Beatmungsgerät nach Anspruch 5, **dadurch gekennzeichnet, daß** der Träger (20) austauschbar ist.

## Claims

1. A carrier (20) comprising moisture and bacterial barriers (21, 22, 24, 25, 26) for a plurality of medical components (10 to 14), the carrier being suitable for connection with a measuring tube (8) provided in the flow path of a ventilator (1), **characterized in that** at least two moisture and bacterial barriers (21, 22, 24, 25, 26) are mounted on the carrier (20) such that a respective barrier can be arranged directly in front of each of the components (10 to 14) when the carrier (20) is applied in the ventilator (1).

2. The carrier (20) for moisture and bacterial barriers according to claim 1, **characterized in that** the carrier (20) has an elongated shape.

3. The carrier (20) for moisture and bacterial barriers according to one of claims 1 and 2, **characterized in that** the peripheral surface of the carrier (20) is provided with a sealing (27) which abuts the measuring tube (8) when the carrier is applied.

4. The carrier (20) for moisture and bacterial barriers according to one of claims 1 to 3, **characterized in that** the barriers (21, 22, 24, 25, 26) are filters and/or diaphragms.

5. A ventilator (1) comprising a measuring tube (8) and a unit (9) in which fixedly mounted components (10 to 14) are arranged, whereby the carrier (20) according to one of claims 1 to 4 is mounted between the measuring tube (8) and the unit (9).

6. The ventilator (1) according to claim 5, **characterized in that** the carrier (20) is exchangeable.

## Revendications

1. Support (20) comprenant des barrières (21, 22, 24, 25, 26) à l'humidité et aux bactéries pour une multiplicité d'éléments (10 à 14) médicaux, en vue du raccordement à un tube (8) de mesure, qui est disposé dans les trajets de flux d'un appareil (1) respiratoire, **caractérisé en ce qu'**il est monté sur le support (20) au moins deux barrières (21, 22, 24, 25, 26) à l'humidité et aux bactéries de façon à ce que celles-ci puissent être disposées respectivement, pour un support (20) appliqué dans l'appareil (1) de respiration, exactement avant chaque élément (10 à 14).

2. Support (20) pour des barrières à l'humidité et aux bactéries suivant la revendication 1, **caractérisé en ce que** le support (20) a une forme qui s'étend en longueur.

3. Support (20) pour des barrières à l'humidité et aux bactéries suivant la revendication 1 ou 2, **caractérisé en ce que** la surface périphérique du support (20) est munie d'une garniture (27) d'étanchéité, qui s'applique au tube (8) de mesure lorsqu'un support (20) est appliqué.

4. Support (20) pour des barrières à l'humidité et aux bactéries suivant l'une des revendications 1 à 3, **caractérisé en ce** les barrières (21, 22, 24, 25, 26) sont des filtres et/ou des membranes.

5. Appareil (1) de respiration, comprenant un tube (8) de mesure et une unité (9) dans laquelle des éléments (10 à 14) sont disposés en étant montés de manière fixe, un support (20) suivant l'une des revendications 1 à 4 étant interposé entre le tube (8) de mesure et l'unité (9).

6. Appareil de respiration suivant la revendication 5, **caractérisé en ce que** le support (20) peut être remplacé.
